# EUROPEAN PATENT SPECIFICATION

(11) **EP 2 359 781 B1**
(45) Date of publication and mention of the grant of the patent: **25.12.2013**
(21) Application number: 09827204.0
(22) Date of filing: 05.11.2009
(51) Int. Cl.: A61F 7/00

(54) **DEVICE FOR PERFORMING BEAUTY, PHYSIOTHERAPY AND HYDROTHERAPY TREATMENT**
VORRICHTUNG ZUR DURCHFÜHRUNG VON SCHÖNHEITS-, PHYSIOTHERAPIE- UND HYDROTHERAPIE-BEHANDLUNGEN
DISPOSITIF POUR MISE EN OEUVRE DE TRAITEMENTS ESTHÉTIQUES, DE PHYSIOTHÉRAPIE ET DE BALNÉOTHÉRAPIE

(30) Priority: 24.11.2008 ES 200803334
(43) Date of publication of application: 24.08.2011
(73) Proprietor: Arb Systems Proyectos Electrónicos, S.L., 28400 Collado Villalba (Madrid) (ES)
(72) Inventor: RAMÍREZ BARRONES, Alberto, E-28400 Collado Villalba (Madrid) (ES); RAMOS BARDI, Alberto, E-43700 El Vendrell (Tarragona) (ES)
(74) Representative: Botella Reyna, Antonio
(86) International application number: PCT/ES2009/000526
(87) International publication number: WO 2010/058043

(56) References cited:
- ES-A1- 2 208 014
- US-A- 5 871 526
- US-A- 5 871 526
- US-A- 5 894 615
- US-A- 5 894 615
- US-A1- 2003 229 385
- US-A1- 2004 068 309
- US-A1- 2004 068 309
- US-A1- 2004 102 826

## Description

### OBJECT OF THE INVENTION

The present invention relates to a device especially designed for performing beauty, physiotherapy and hydrotherapy treatments based on contrasting applications on any part of the body, locally or generally and at high speed, wherein the patient does not come into contact with vapours or fluids.

Some of the applications or treatments which can be carried out using the equipment of the invention are:
Facial: moisturisation, oxygenation, nutrition, reaffirmation, facial hygiene, facial exfoliation, facial rejuvenation, anti-wrinkle, among others:
   - Body: anti-cellulite, reaffirming treatment, circulatory treatment, drainage, reducing treatment, body exfoliation, body moisturising, body rejuvenation, leg relaxation and beauty, among others.
   - Shape-up and hydrotherapy: anti-stress treatment, circulatory treatment, remineralising treatment, detoxification treatment, etc.
   - Mud therapy with contrasting applications: mud facial mask, mud wrap, mud packs, etc.
   - Physiotherapy: treatment of contractions, muscular pain, etc., among many others.

### BACKGROUND OF THE INVENTION

At present, all those treatments essentially based on contrasting applications are performed using:
- As heating methods:
   ○ Sauna (dry heat).
   ○ Turkish (heating by immersion of the patient in a room full of steam).
   ○ Hot water (immersion in a bathtub or application of jets) .
- As cooling methods:
   ○ Cold water: by application of jets, shower or immersion in a bathtub.
   ○ Cold room: introduction of the patient in a room at low temperature.

Some methods as disclosed in U.S. Patent 5,871,526 use Peltier cells for cold and hot applications, with essential differences with respect to the present invention, among which the following must be highlighted:
o
   1. Contrasting applications are performed by placing the metal blocks that house the Peltier Effect modules directly onto the patient, which implies a difficult and uncomfortable adaptation thereto by the user, to which we must add the fact that the fluid does not circulate at the adequate temperature, i.e. it is not previously treated, but rather directly treated in said blocks.
o The use of hot/ice thermal blankets in physiotherapy is known, the application surface of which is either very limited, in the order of 150 cm², (localised treatments), or up to surfaces in the order of 20,000 cm² or more (the patient's whole body).

In general, the current methods for performing treatments using contrasting applications have drawbacks such as:
- Contrasting is sudden, passing from heat to cold and vice versa without previously preparing the patient and without gradual temperature control (methods that use immersion, jets, etc.).
- Contrasting is not gradual throughout the body, but rather the temperature contrast occurs simultaneously throughout the body. It must be taken into account that not all body parts support sudden temperature changes in the same manner, particularly cold applications.
- Contrasting is uncomfortable and unpleasant, in such a manner that contact with cold water is not tolerated well by the patient.
- The treatments are general and cannot be performed locally or, if applicable, are focused on a single point, whereupon different points cannot be treated simultaneously with different treatments.

### DESCRIPTION OF THE INVENTION

The object of the invention being presented is to overcome the drawbacks of the state of the art by means of equipment capable of performing an unspecified number of treatments, on any part of the body, locally or generally (on surfaces ranging from 12 cm² to the whole body), at high speed, wherein the patient does not come into contact with vapours and/or water, either manually (the user determines all the work parameters of the equipment: from the area or areas to be treated, to the number of contrasting applications, the temperature of each of the applications, application frequency, etc.), or automatically with pre-programmed treatments loaded into the data base of the equipment, such as beauty, hydrotherapy and physiotherapy treatments, etc., essentially using contrasting applications to this end. The patient receives the contrasting applications by means of so-called contrast modules, which are made of impermeable, flexible and heat-conducting material, are designed to suit each of the areas of the patient to be treated and are disposed by way of wrapping or covering in direct contact therewith: arms, legs, abdomen, back, face, etc.

Fluid circulates through the contrast modules, having previously been heated or cooled to the appropriate temperature using the thermostat control module and, without coming into direct contact with the patient, transmits the different temperatures therethrough (cold and hot applications). The equipment includes, for operation thereof, a thermostat control module with one, two, three or more independent circuits. Each of these circuits has a heat module (heater module) with heating resistors and a cold module (high-performance cooling module) based on Peltier Effect cells. Both the heat and cold module may or may not be common to the different independent circuits. The combination of both systems, together with the control and programming module, enable contrasting (passage from hot application to cold application and vice versa) to take place in an extremely short period of time (seconds), said peculiarity providing a very high level of effectiveness in the treatments.

The invention is capable of controlling the area or areas to be treated with the contrasting applications (contrasting treatments can be performed on different areas, at different temperatures, duration, frequency, etc. and simultaneously). The equipment is capable of controlling the duration of each of the treatments independently, in addition to the duration of each of the contrasting applications (duration of the hot and cold applications). Likewise, it is capable of controlling the frequency between applications, in addition to the temperature of each application, independently in the different areas to be treated and in ranges which can be selected by the user which range, for cold application, from 2°C to 35°C, depending on the treatment and/or area to be treated and, for hot application, from 35°C to 50°C, depending on the treatment and/or area to be treated. The equipment is capable of producing the contrast, passing from heat to cold and vice versa in an extremely short period of time (seconds), and is capable of doing so progressively, starting the hot or cold application, for example, on the legs and ascending progressively up the back, abdomen and arms. The invention is also capable of performing localised contrasting treatments and applications: leg or legs, and/or back, and/or abdomen, and/or face, etc.; likewise, it is capable of combining any of these areas based on the user's criteria.

To this end, the device of the invention consists of a casing, housing in its interior:
- A control and programming module (control electronics), which in turn incorporates:
- An alarm module.
- A music module.
- A thermostat control module.
- Fluid inlets.
- One outlet for a touch screen.
- One outlet for an external printer.
- A thermostat control module having one, two, three or more independent heat/cold circuits. A complete cold circuit and a complete heat circuit. Each of these circuits has a heat module (heater module) based on heating resistors (boiler), and a cold module (high-performance cooling module) based on Peltier Effect cells.

The temperature ranges for each of the applications can be adjusted according to the treatment, the area to be treated, etc. between margins ranging from:
■ Cold application: between 2°C and 35°C.
■ Hot application: between 35°C and 50°C.
   - Hot fluid circuits: The basic heat circuit is formed by a reservoir for the hot fluid, a boiler (heating resistors), at least two temperature probes, one disposed at the outlet of the boiler and another in front of the inlet of the contrast modules, a pump for impelling the fluid throughout the circuit, a radiator for lowering the temperature of the fluid flowing out of the contrast modules, in such a manner as to reduce the thermal impact and lower the temperature to around 25°C/30°C, appropriate for flowing into the boiler, where it will be adjusted to the temperature programmed for the next treatment, un unspecified number of electrovalves and a discharge pump for removing the water from the contrast modules, prior to filling with the cold fluid, thereby optimising the contrast by reducing temperature losses to a maximum.

The complete heat circuit has the following common elements in the different independent circuits: the hot fluid reservoir, the radiator and the pump, and incorporates in each of the independent circuits: a heating resistor module (boiler) which may or may not be the same as that used in the different cold circuits, a discharge pump which may or not be common to that used in the different cold circuits, in addition to independent temperature probes per circuit, which also may or may not be common to those used in the different cold circuits.
- Cold fluid circuit: the basic cold circuit is formed by a reservoir for cold water, a pump for impelling the fluid through the cold circuit, a boiler (heating resistors) to help control the temperature of the cold circuit, at least two temperature probes, one disposed inside the cold fluid reservoir and another at the inlet of the contrast modules which is common to the two circuits (heat/cold), a high-performance cooling module based on Peltier Effect cells, the operation of which is explained later in detail, an unspecified number of electrovalves and a discharge pump which is common to the two circuits (heat/cold).

The complete cold circuit has elements common to the different independent circuits: the cold fluid reservoir, the high-performance cooling module and the pump, incorporating in each of the independent circuits: a heating resistor module, (boiler), which may or may not be the same as that used by the different heat circuits, a discharge pump, which may or may not be common to that used by the different heat circuits, in addition to independent temperature probes per circuit which also may or may not be common to those used by the different heat circuits.

The invention may or may not have one, two, three or more independent temperature circuits, each of which is controlled independently by the control and programming module. They all use the cold/hot water reservoirs, in addition to the cooling module. And each of the three circuits has, independently, in order to control and reach the programmed temperature of a boiler, (heating resistors), and of the corresponding temperature probes, both in the cold and heat circuit, in addition to independent pumps, both filling and discharge, for the cold and heat circuits.

The high-performance cooling module is based on thermoelectric systems, Peltier cells and, while forming part of the equipment being protected as a whole, the use of this module for cooling and/heating fluids for applications in aesthetic, beauty, physiotherapy and/or hydrotherapy treatments in itself represents an innovation aimed at overcoming the drawbacks of the state of the art, by means of a device capable of cooling a circulating fluid, whether air or liquid, for subsequent use thereof in cooling and/or heating any element or device, in this case the so-called contrast modules and their application in beauty, physiotherapy and/or hydrotherapy treatments.

Additionally, a control and programming module (control electronics) is disposed to control the different operating parameters of the equipment: cold and heat temperatures, number of contrasting applications, frequency of the applications, areas to be treated in each treatment, etc. In addition to providing the user with a tool that is flexible, powerful and extraordinarily accurate and safe. Which, by means of a user-friendly touch screen, allows the user, on one hand, to establish and control the different operating parameters and programmes of the equipment and, on the other, to visualise the necessary data to keep track of the real status of the equipment and treatment at all times. The control electronics incorporates the necessary elements to control the different elements of the equipment: pumps, electrovalves, temperature probes, heating resistors, cooling module, etc. Said module is aided by a touch screen, which is the interface between the user and the equipment, containing the on/off switch of the equipment, the controls for the equipment to work in manual or automatic mode, the controls for establishing the type of treatment to be performed, the modifications we want to make thereto to adapt it to the patient, the number of contrasting applications, the temperature of each, the duration of each application, etc. Likewise, through this screen, the user is capable of learning the status of the equipment, the activated alarms, if any, the remaining time in the treatment, the temperature of the fluid we are applying in real time, etc. On the other hand, the touch screen is used by the user by means of a touch keypad displayed thereon to introduce the patient's data in the data base implemented to this end or to access the patient's record, etc.

The device is complemented by a protection and alarms module that provides the equipment with the necessary safety mechanisms in case of possible eventualities, which envisages temperature alarms, heater disconnection system in case of equipment malfunction, pressure drop alarm in any of the contrast modules, low liquid level alarm and end-of-treatment alarm, in addition to an alarm indicating excessive cold or hot application or improper use of the equipment.

With regard to the contrast modules, these modules, which have different dimensions depending on the area or areas to be treated, cover the patient's treatment surface or surfaces and are made of impermeable, flexible and heat-conducting material, wherethrough the fluid circulates at the preset temperature. The contrast modules are in contact with the patient (applied over a disposable plastic film for individual use which is disposed between the contrast modules and the patient's skin for hygiene reasons). The contrast modules are in charge of transmitting the contrasting applications to the patient. Said modules are connected to the thermostat control module and, where applicable, therebetween, by the fluid supply and return pipes. The contrast modules which can be used by the invention have different sizes and shapes and are designed to suit the needs and surface to be treated in each case.

The invention envisages the inclusion of a direct contrasting applicator, consisting of a manual device, especially designed for localised treatments in small areas (no more than 12 cm²). This device may have different shapes and sizes depending on the surface to be treated. With regard to its constituent material, any material having sufficient heat-conducting characteristics may be used.

The equipment may be connected to a mains electricity supply with a voltage comprised between 100v and 240v and having a frequency between 47Hz and 62Hz, which facilitates use thereof without any type of modification in most countries around the world.

The invention has two basic operating modes: manual and automatic.

In both manual and automatic operating mode, the professional may, among other functions, select one of the standard treatments: beauty (facial and/or body), shape-up, hydrotherapy, etc. and modify it in order to adapt it to the needs of the patient to be treated, retrieve necessary data from the data base and the treatment history of a specific patient, either to verify said information or modify or add to it, or use the "direct contrasting applicator" simultaneously with the treatment being performed.

Automatic operation is aided by control electronics designed to control and provide the professional with a tool that is flexible, powerful and extraordinarily accurate and safe.

Therefore, the following advantages are derived from the previously described structure:
- One of the substantial advantages of the designed equipment is its capacity for performing contrasting, passing from cold to hot application and vice versa in seconds and the control that it provides over said applications at all times, both in relation to application time and application temperature and frequency of the different applications, differentiating these based on the area or areas to be treated.
- Use of the contrast modules with sizes adapted to the area or surface to be treated allows, on one hand, treatment intensification (more intense and prolonged cold and hot applications), depending on the area to be treated or the treatment to be performed and, on the other, localised cold application that is much more tolerable by the patient than whole-body cold impact, which causes a greater impact and is much less "commercial."
- With the designed equipment, the contrast or change in temperature is progressive, starting in the legs and gradually approaching the abdomen, back, etc. sequentially and progressively, allowing better adaptation to the contrast and making it more pleasant and effective.
- The temperature is adapted in accordance with the area to be treated, in terms of both application time and application temperature.
- Another essential advantage is that, in both local and general treatments, the equipment allows the professional, whether beautician or physiotherapist, etc., to combine the contrasting applications with the necessary manual therapy to enhance and optimise the results of the treatment to be performed, whether beauty, rehabilitation, etc., which can be done by alternating manual therapy and contrasting in the required number and with the frequency and duration deemed necessary.
- Total personalisation of the treatment is another of the major advantages of the equipment; in fact it is possible to personalise both the number of applications and the duration and frequency of the applications, the temperature of each application, etc. It is possible to design adapted treatments personalised for each patient, in addition to preserving a record of each customer, etc.
- Another advantage is ease of use and programming, not only due to having incorporated a powerful microprocessor that provides extraordinary flexibility, but also because the equipment has a powerful memory that stores a large number of standard treatments which the professional can apply directly or use as a base for those they design for each client. This ease of use enables any beautician, physiotherapist, etc., regardless of their degree of experience, to perform enormously effective treatments on their patients.
- Another major advantage of the equipment is that the patient does not get wet; in fact, the equipment provides the benefits of water (contrasting) without the drawbacks of being in direct contact therewith.

### DESCRIPTION OF THE DRAWINGS

In order to complement the description being made and with the object of helping to better understand the characteristics of the invention, in accordance with a preferred example of embodiment thereof, a set of drawings is attached as an integral part of said description where, in an illustrative and non-limiting manner, the following has been represented:
- Fig. 1: shows a general block diagram of the assembly, which constitutes the device for performing beauty, physiotherapy and hydrotherapy treatments of the invention;
- Fig. 2: shows a general elevational and side view of the equipment, which includes the outer casing housed in the lower part of a bed, with the different previously described elements;

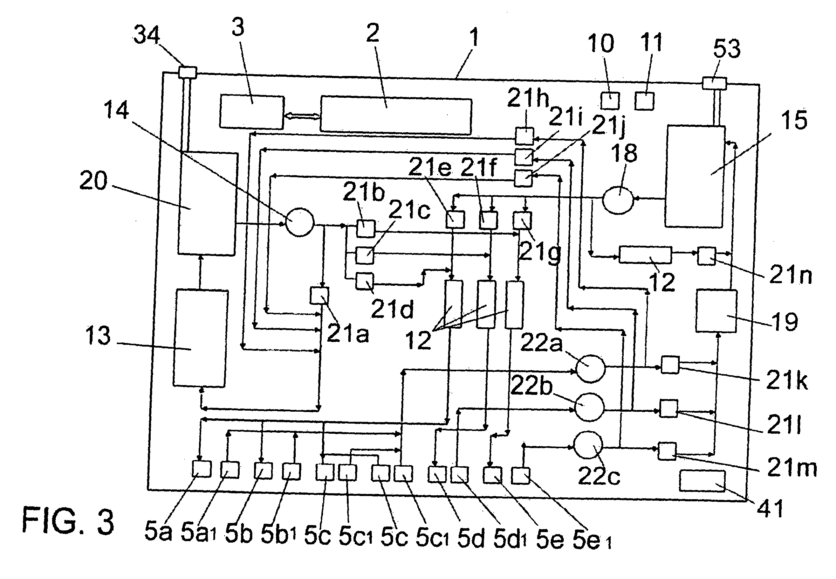

- Fig. 3: shows a block diagram where the distribution and direction of flow of the fluids can be observed;

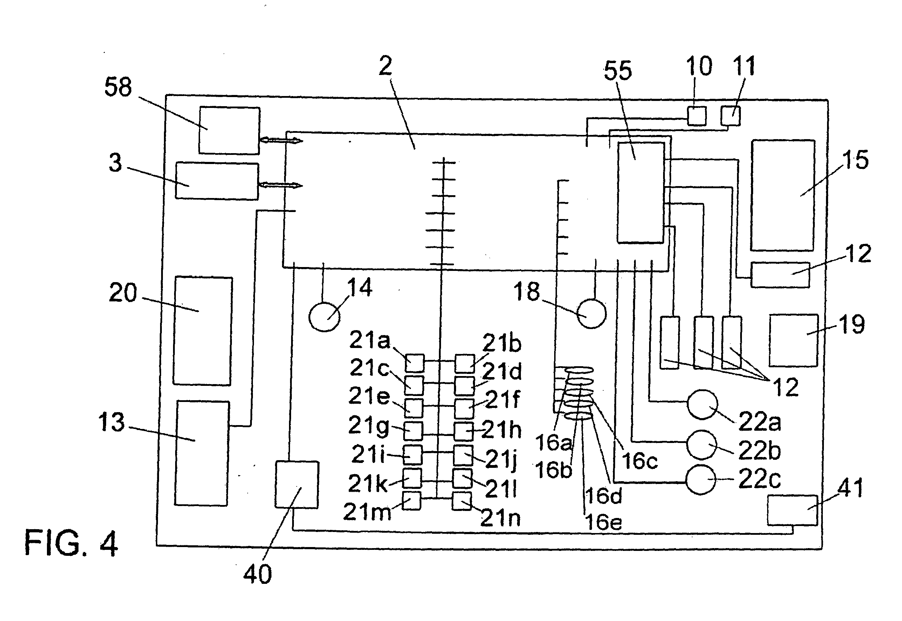

- Fig. 4: shows a block diagram of the electrical and control connections of the equipment of the preceding figures;
- Fig. 5: shows a plan view of the cooling module;
- Fig. 6: shows a schematic detailed view of the circulator unit of the cooling module;
- Fig. 7: shows frontal elevational, right side, left side and plan views of a circulator in partial cross-section;
- Fig. 8: shows a block diagram of the different elements that participate in the basic heat/cold circuit;
- Fig. 9: shows a schematic view of the standard contrast module;
- Fig. 10: shows a longitudinal sectional view of the direct contrasting applicator; and
- Fig. 11: shows a block diagram of the operation and elements that participate in the complete heat/cold circuit.

### PREFERRED EMBODIMENT OF THE INVENTION

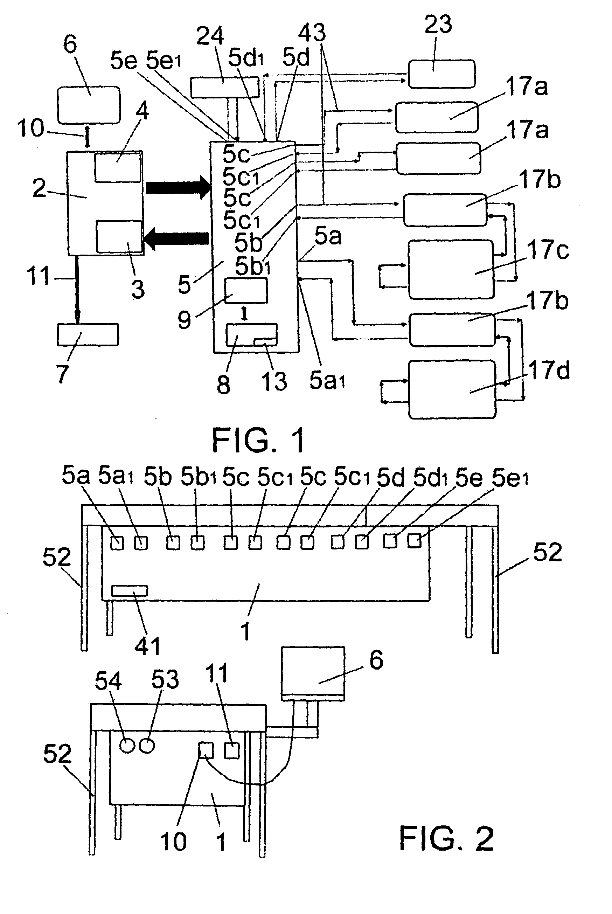

In light of the preceding figures, particularly figures 1 and 2, it can be observed how the device of the invention consists of a casing (1) which houses a control and programming module (2), which in turn includes an alarms module (3) and a music module (4), an element associated to a thermostat control module (5) with its corresponding fluid inlets (5a, 5a1, 5b, 5b1, 5c, 5c1, 5d, 5d1, 5e and 5e1), said control and programming module (2) including an input (10) for a touch screen (6) and a second input (1) for an external printer (7).

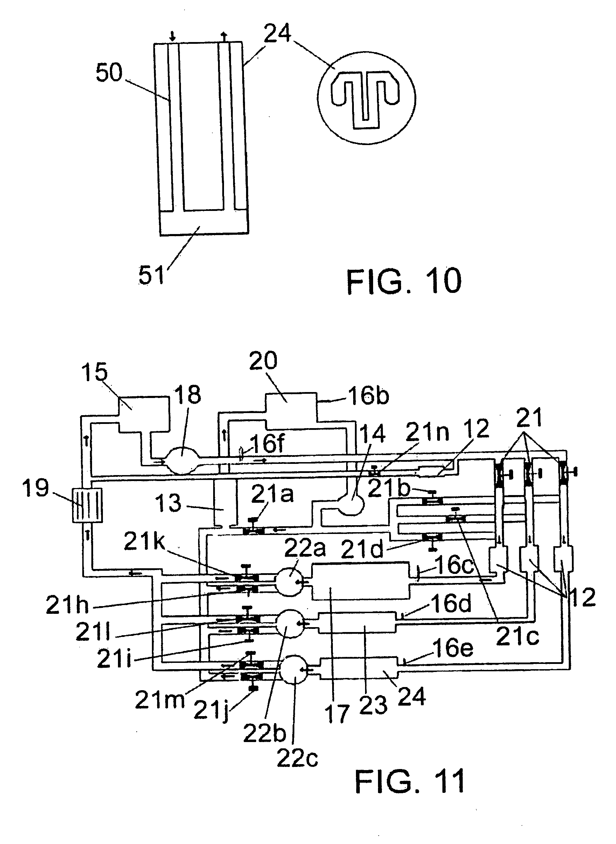

For its part, the thermostat control module (5) includes one, two, three or more independent cold (8)/heat (9) circuits, those shown in detail in figure 11. Each of these circuits has a heat module (heater module) based on heating resistors (12) and a cold module (13) (high-performance cooling module) based on Peltier Effect cells, associated to the corresponding cold pump (14).

The temperature ranges for each of the applications can be adjusted according to the treatment, the area to be treated, etc., within the following margins:
■ Cold application: between 2°C and 35°C.
■ Hot application: between 35°C and 50°C.

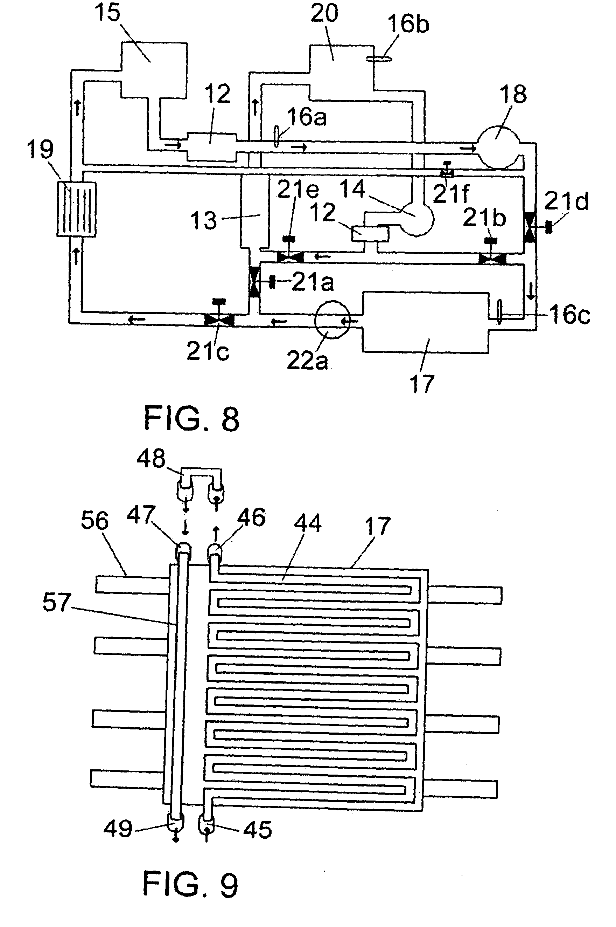

With regard to the hot fluid circuits corresponding to figures 8 and 11, the basic heat circuit (figure 8) is formed by a reservoir for the hot fluid (15), a heating a heating resistor (12), three temperature probes (16a, 16b, 16c), one at the outlet of the heating resistor (12), another in front of the inlet of the contrast modules (17) and a third optional probe inside the cold water reservoir (20), a heat pump (18) for impelling the fluid throughout the circuit, a radiator (19) for lowering the temperature of the fluid at the outlet of the contrast modules, in such a manner as to reduce the first thermal impact and lower the temperature to around 25°C/30°C, appropriate for entering the heating resistor (12) or boiler, where it will be adjusted to the temperature programmed for the next treatment, an unspecified number of electrovalves (21) and a discharge pump (22a) for emptying the water from the contrast modules before refilling these with the cold fluid, thereby optimising the contrast and reducing temperature losses to a maximum.

For its part, the complete heat circuit (figure 11) has the following independent circuits by way of common elements: the hot fluid reservoir (15), the radiator (19) and the heat pump (18), and incorporates in each of the independent circuits: a heating resistor module (12) which may or may not be the same as that used by the different cold circuits, a discharge pump (22a) which may or may not be common to that used by the different cold circuits, and independent temperature probes (16a, 16b, 16c) per circuit, which also may or may not common to those used by the different cold circuits.

With regard to the cold fluid circuit, in the chosen example of embodiment it is associated with the heat circuit in order to avoid duplicity of the elements, but it could nevertheless consist of an independent circuit without affecting the essence of the invention, in such a manner that it is formed by a reservoir for cold water (20), a cold pump (14) for impelling the fluid through the cold circuit, a heating resistor (12) or boiler to help control the temperature, coinciding with those previously described, on being common for the two circuits (heat/cold), a high-performance cooling module (13) based on Peltier cells, the operation of which is explained later in detail, an unspecified number of electrovalves (21) and a discharge pump (22a), also common to the two circuits (heat/cold).

Going on to analyse the complete cold circuit, the different independent circuits are its common elements: the cold fluid reservoir (20) the high-performance cooling module (13) and the cold pump (14), and incorporates in each of the independent circuits: a heating resistor module (12) which may or may not be the same as that used by the different heat circuits, a discharge pump (22a) which may or not be common to that used by the different heat circuits, and independent temperature probes per circuit which may or may not be common to those used by the different heat circuits.

Optionally, the high-performance cooling module (13) is substituted for a conventional cold compressor.

The basic heat circuit, that shown in figure 8, functions as follows: when the thermostat control module (5) receives the instruction to heat and upon previously emptying the circuit using the discharge pump (22a), valves (21b and 21a) are closed and valve (21e) is opened, the cold pump (14) continues working, impelling the cold fluid in a closed circuit through the cooling module (13) to maintain the temperature in the cold reservoir (20) at the programmed temperature. Valve (21f) is closed to prevent spillback of hot fluid and valves (21d) and (21c) are opened, actuating the heat pump (18) in such a manner that the fluid circulates through the circuit, is heated on passing through the heating resistors (12) at the programmed temperature, circulates through the contrast modules (17) at the expected temperature and, on flowing out of said modules, flows through the radiator (19), which reduces the first thermal impact, lowering the temperature of the fluid to around 25°C/30°C, in such a manner that it is in a condition to be treated by the heater resistor (12) again in order to be raised to the temperature programmed for the next circulation of fluid through the contrast modules (17). The equipment continues in this state during the time programmed for hot application. Upon conclusion of hot application and before cold application, valve (21d) is closed, the heat pump (18) is stopped and the discharge pump (22a) is started up, emptying the water from the contrast modules (17) and depositing it in the hot water reservoir (15). When the contrast modules (17) have been emptied, valve (21c) is closed, the discharge pump (22a) is stopped and the cooling process starts.

For its part, the basic cold circuit functions as follows: Once the hot fluid has been emptied, the discharge pump (22a) stops, valve (21c) is closed and valve (21e) is closed to prevent recirculation of the cold fluid. Valves (21a) and (21b) are opened and the cold pump (14) continues working, emptying the fluid from the cold tank into the contrast modules (17) in a matter of seconds. The temperature of the cold fluid is controlled by the heating resistor (12) disposed at the outlet of the cold pump (14), which adapts it to the programmed temperature before reaching the contrast modules (17). The cold fluid that flows through the cooling module and reaches the contrast modules is thus recirculated, passing through the heating resistor (12) that controls the temperature and adapts it to the temperature programmed for cold application. Once the time of the cold application has expired, the cold pump (14) is disconnected and valve (21b) closed to avoid turbulences and recirculations in the contrast module (17) emptying process, and the discharge pump (22a) is activated, which through valve (21a) will empty the cold fluid from the contrast modules into the cold water reservoir (15). Once the contrast modules have been emptied, the discharge pump (22a) is disconnected, valve (21a) is closed, valve (21e) is opened and the cold pump (14) is started up to start recirculating the cold fluid through the cooling module (13), in a closed circuit that will lower the preparation temperature for the next cold application. At this point, two cases can occur: one where a new hot application starts, in which case we would restart the heat circuit operating process, and another where the treatment will have concluded, whereupon the equipment would remain in the indicated state, i.e. recirculating the cold fluid and maintaining its temperature within the programmed margins, waiting for a new treatment to start, unless the equipment is disconnected, in which case all the devices of the equipment are disconnected and go into sleep mode.

As mentioned earlier, the invention may optionally have independent temperature circuits: the invention may have one, two, three or more independent temperature circuits. For descriptive purposes, the invention has been embodied with three temperature circuits, each controlled independently by the control and programming module (2).

Therefore, said circuits may also be associated to the contrast module (17), to a facial module (23) or to a direct contrasting applicator (24).

They all use, as common elements, the cold/hot water reservoirs, in addition to the cooling module. And each of the three circuits has an independent heating resistor (12) to control and reach the programmed temperature, as well as the corresponding temperature probes (16c, 16d, 16e) both in the cold circuit and heat circuit, in addition to independent filling and discharge pumps (22a, 22b and 22c) for the cold circuit and for the heat circuit.

With regard to the high-performance cooling module (13), it is based on thermoelectric systems, Peltier cells and, while forming part of the equipment being protected as a whole, the use of this module for cooling and/or heating fluids for applications in aesthetic, beauty, physiotherapy and/or hydrotherapy treatments represents, in itself, an innovation which has the object of overcoming the drawbacks of the state of the art, by means of a device capable of cooling a circulating fluid, whether air or liquid, for subsequent use thereof in cooling and/or heating any element or device, in this case in the so-called contrast modules and its application in beauty, physiotherapy and/or hydrotherapy treatments.

The objective is achieved by developing a high-performance cold generator based on Peltier Effect cells.

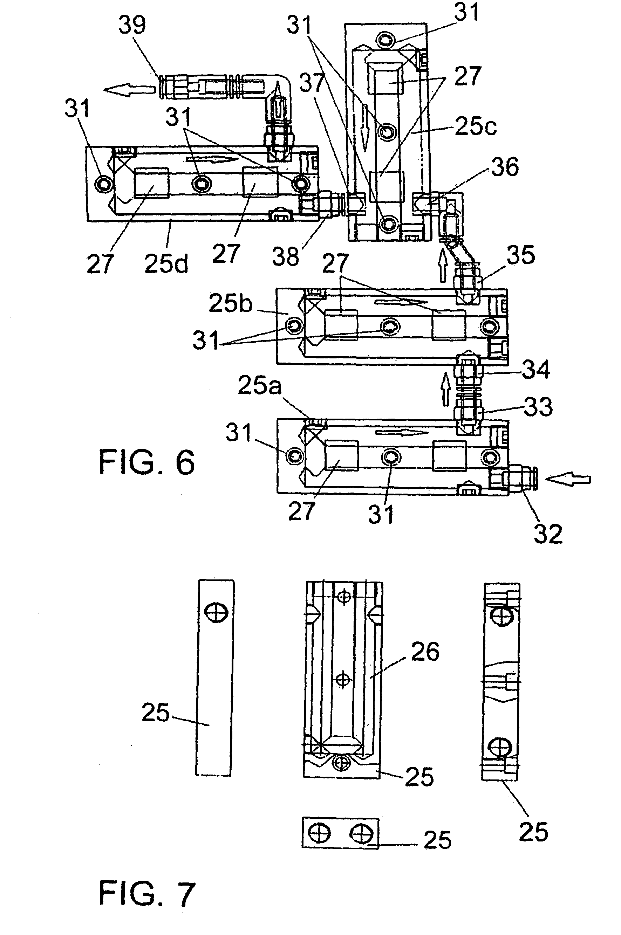

On one hand, the device is composed of a "circulator unit", that shown in figure 6, wherethrough the fluid to be cooled circulates and is cooled by Peltier Effect cells. The circulator unit is formed at least by one circulator element (25), made from heat-conducting material (aluminium is an especially appropriate material on account of its heat-conducting characteristics and ease with which it is heated and cooled).

The cooling module (13) of the invention may have one, two, three, four or more circulator elements (25a, 25b, 25c and 25d), such as those mentioned earlier, joined together as shown in the aforementioned figure 6. For descriptive purposes, the assembly of the invention has been configured using four independent circulator modules joined together (25a, 25b, 25c and 25d), made of heat-conducting material and machined internally into a spiral shape (26), as shown in figure 7, in such a manner that the circulating fluid to be cooled flows through the spiral of each of the circulator elements (25a, 25b, 25c and 25d), following a path and patterns that will be explained later in detail.

In order to cool each of the circulator elements, Peltier cells (27) are used, placed in direct contact therewith and fixed in the manner explained later. Given the characteristic of Peltier cells of generating heat, based on the polarity of the direct current supplied thereto, on one of their sides and cold on the other and vice versa by changing the polarity of the direct current supplied thereto. The invention being presented is only capable of cooling, heating or cooling and heating, using the adequate means that will allow a change in polarity of the current supplied to the Peltier cells (27) incorporated in the control and programming module (2) of the equipment. Each of the circulator elements (25) comprises a diversity of Peltier cell units (27), which may be conventional or double. For descriptive purposes, the assembly has been configured using four double Peltier cells (27) per each of the circulating elements, two on each of its sides, has been used. On their cold side, these are installed two by two on each of the sides of the four circulator modules (25 that form the "circulator unit." These may be joined to the aforementioned circulators using separators (28) made of heat-conducting material, 42x42 mm in size and 10 mm thick, in such a manner that the Peltier cells (27) are joined to said separators by their cold side using heat-conducting paste and these in turn, also using heat-conducting paste, to their respective circulators, whereon they radiate cold for the purpose of cooling the circulating fluid. In the event of not using the aforementioned separators, the Peltier cells (27) will be joined directly by heat-conducting paste to their respective circulators. On the other hand, the Peltier cells (27) will be joined by their hot side to their respective radiators (19) using heat-conducting paste. The system for fixing the Peltier cells, on one hand, to their corresponding separator, if any, and to the circulator and, on the other, to the heat exchanger, is composed of three screws (29) per circulator, in such a manner that they pass through the corresponding circulator, using isolators that prevent the screws from coming into contact with the circulator, thereby avoiding temperature transfer and cold loss in the unit. They pass through the circulator by means of three holes (31) made therein and are fixed by means of nuts to the base of the respective radiators so that, in a sandwich-like manner, they exert pressure on and fix their respective Peltier cells, separators and the circulator remaining in the centre between one radiator and another, ensuring fixation of the unit and perfect contact between the Peltier cells and the corresponding circulator, on one hand, and with the high-performance heat exchanger (corresponding blade radiator) on the other, guaranteeing maximum cold transfer towards the circulator and adequate cooling of each of the Peltier cells on their hot side by establishing perfect contact with their corresponding radiator.

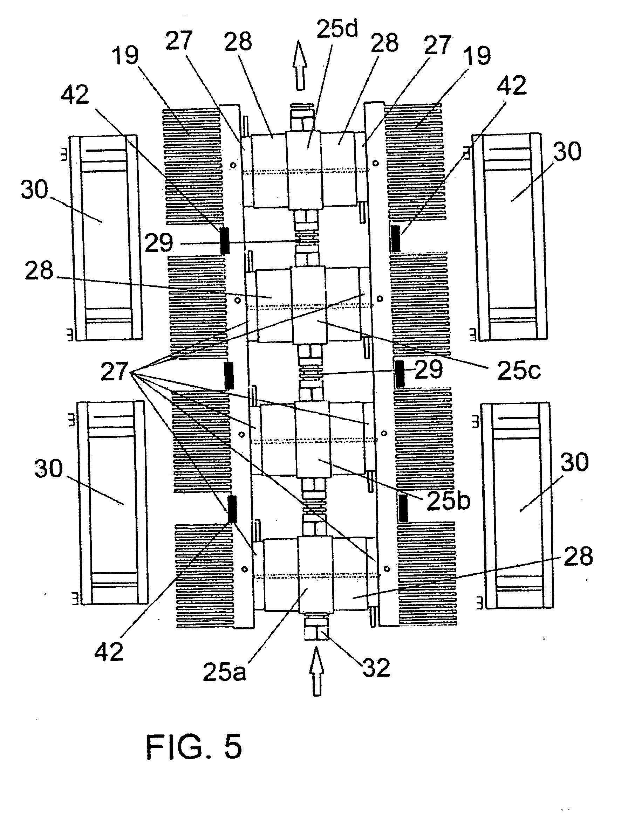

As explained earlier, the Peltier cells (27), on their hot side, are joined, also using heat-conducting paste, to the high-performance heat exchangers or radiators (19). These are formed by blade radiators and fans (30), one per radiator, which by forcing the circulation of air evacuate the hot air generated by the Peltier cells to the exterior of the unit, which is collected by the blade radiators. The invention contains one, two, three, four or more high-performance heat exchangers, each of which is formed by a radiator (19) and a fan (30). For descriptive purposes, the assembly of the invention has been configured using four of the aforementioned heat exchangers, being interdependent, as shown in figure 5, in such a manner that each fan impels the air so that it circulates through the corresponding blade radiator and cools its radiator, in such a manner that the air of the different exchangers does not become mixed, thereby preventing the air turbulences that would reduce the effectiveness of the Peltier cells.

Optionally, the so-called heat exchangers, consisting of radiators (16) and fans (30), may be substituted for a fluid-based cooling system.

The distribution of the unit formed by the sixteen double Peltier cells (27) joined, as explained earlier, back to back on each of the sides of the circulator modules (25a, 25b, 25c and 25d), (four Peltier cells per circulator), which form the "circulator unit", plus the four high-performance heat exchangers, formed by the blade radiators (19) and their respective fans (30), is the following: as inferred from observation of figure 5, the unit formed by the circulators (25a and 25b), in addition to the eight Peltier cells (27) installed therein, are joined in the aforementioned manner to the same number of heat exchangers (radiators + fans). Likewise, the unit formed by the circulators (25c and 25d) and the eight Peltier cells (27) installed therein, are joined, in the previously explained manner, to the same number of exchangers, formed by a radiator and fan.

Coming back to the cooling of the circulating fluid: on one hand we have the "circulator unit" shown in figure 6, which is cooled by the sixteen double Peltier Effect modules (27) installed therein, which in turn are cooled and maintained at optimum work temperature margins thanks to the four heat exchangers (radiators + fans), while the fluid to be treated penetrates by means of a joint nut (32) and pipe, of the circulator (25a) which may or may not incorporate a tubulator (cross-shaped device installed in the interior of a circulator module, throughout the conduit wherethrough the fluid circulates). This device may or may not be installed in the circulators (25a, 25b, 25c and 25d), in such a manner that if it is installed it forces the fluid to circulate rotating, thereby increasing its level of friction against the walls of the conduit wherethrough it flows towards the circulator modules, thereby increasing the contact between the element to be treated and the cooling device (circulator), conferring a higher level of effectiveness to the unit. Once inside the circulator (25a), the fluid flows through the interior thereof and out of said circulator through the outlet (33), also using a joint nut and pipe. The fluid to be cooled is made to circulate from said outlet to the outlet (34) located in the circulator (25b). The fluid penetrates the circulator, flowing through the interior thereof, using or not using the corresponding tubulator, until flowing out through the outlet (35). Also using a joint nut and pipe, the fluid to be cooled is made to flow from said outlet to the outlet (36) of the circulator (25c). The fluid penetrates in said circulator and, using or not using the tubulator, flows therethrough until flowing out thereof through the outlet (37). Also using a joint nut and pipe, the fluid to be cooled is made to flow from said outlet to the outlet (38) of the circulator (25d). The fluid penetrates in said circulator and flows therethrough until flowing out through the outlet (39). At this point, in order to extract the already cooled fluid from the unit and use it in the corresponding application (in this case for cooling the contrast modules), a system of pipes and joint nut is used wherewith the device is connected to the corresponding fluid circuit, that shown in figure 8.

When having to refrigerate fluids below 0°C, in the case of air it must previously have been dehydrated or, in the case of liquids, these must be of the "anti-freeze" type and support the temperatures to which they will be subjected without freezing.

In terms of insulation of the unit: the unit formed by the circulators, four in this case, in addition to the connections therebetween, which are made using a joint nut and pipes, and the corresponding heat exchangers, are housed inside a box made of sheet metal or any other material and insulated from the exterior and therebetween using an insulation material such as, for example, polyurethane foam, in such a manner that the cold losses of the unit are practically negligible.

With regard to controlling the temperature of the fluid that flows out of the device: this is achieved using a temperature probe (16b), and the corresponding control module.

For its part, and in terms of the external power supply and electric connections of the high-performance cooling module, the power supplied to the unit, i.e. the Peltier Effect modules (27) and the unit cooling fans (30) is controlled by the control and programming module (1) and a power source (40) incorporated in the unit, which can be 24V, 13A or 12V, 26A or 36V, 9^{a}, associated with a power socket (41). The invention is prepared for connection to both an independent power source such as those mentioned earlier and to the control and programming module of the equipment.

The invention incorporates a mechanical heat disconnection system in case of equipment malfunction. This system is totally independent from the operation of the control electronics installed in the equipment. If for any reason the equipment electronics does not react to an increase in temperature with the corresponding alarm and the temperature continues to increase, the equipment incorporates four independent safety thermal limit controllers (42), one for each of the four heat exchangers, automatically resettable, which do not allow the Peltier cells (27) and the unit as a whole to reach temperature levels higher than those preset in-factory as safety levels, opening the feed circuit thereof in the event of an uncontrolled temperature increase. The thermostats recover their work position and re-establish the supply to the Peltier cells as soon as the temperature returns to its normal values.

Going back to the control and programming module (2) again, it is designed to control the different operating parameters of the unit: cold and heat temperatures, number of contrasting applications, frequency of the applications, areas to be treated in each treatment, etc., in addition to providing the user with a tool that is flexible, powerful and extraordinarily accurate and safe which, by means of a user-friendly touch screen (6), allows the user, on one hand, to establish and control the different operating parameters and programmes of the device and, on the other, to visualise the necessary data to keep track of the real status of the equipment and treatment at all times. The control electronics incorporates the necessary elements to control the different elements of the equipment: pumps, electrovalves, temperature probes, heating resistors, cooling module, etc.

The touch screen (6) constitutes the interface between the user and the equipment and contains the start/stop push button of the equipment, the controls for the equipment to work in manual or automatic mode, the controls for establishing the type of treatment to be used, the modifications we wish to make thereto to adapt it to the patient, the number of contrasting applications, the temperature of each, the duration of each application, etc. Likewise, through this screen the user is capable of learning the status of the equipment, the activated alarms, if any, the remaining time in the treatment, the temperature of the fluid we are applying in real time, etc. On the other hand, the touch screen is used by the user by means of a touch keypad displayed thereon, to introduce the patient's data in the data base implemented to this end or to access the patient's record, etc.

The protection and alarms module (3) provides the equipment with the necessary safety mechanisms in case of possible eventualities, such as the over-temperature alarm. If the temperature of the thermostat control module (5) or of the circulating fluid exceeds the established safety parameters, the over-temperature alarm would become activated, whereupon it gives off an intermittent beep, sends a message to the screen and alerts of the incident while cutting off the current to the heating components.

This alarm will remain activated until it is deactivated manually or until the temperature of the module reaches normal values, in which case it will be deactivated on its own.

This alarm, together with the following alarm, has maximum priority, due to which it will prevail over any other alarm.

Likewise, the incorporation of a heat disconnection system due to equipment malfunction has been envisaged, in such a manner that, if for any reason, the equipment electronics does not react to an increase in temperature in the cooling module (13) with the corresponding alarm and the temperature continues to increase, the equipment incorporates four independent safety thermal limit controllers (42), one for each of the four heat exchangers, automatically resettable, which do not allow the heat-generating elements to reach temperature levels higher than those preset in-factory as safety levels, opening the feed circuit thereof in the event of an uncontrolled temperature increase. Operation of these thermostats is totally independent to the equipment electronics. The thermostats recover their work position and re-establish the supply to the heating modules as soon as the temperature returns to its normal values. This eventuality, as in the previous case, would activate the over-temperature alarm, sending a message to the screen with an intermittent beep.

Complementarily, the incorporation of a pressure drop alarm in any of the contrast modules has been envisaged. In the event that the system were to detect a lack of pressure in any of the contrast modules, whether due to loss of fluid or any other eventuality, the unit will activate a "pressure drop" alarm, sending a message to the monitor and giving off an intermittent beep while isolating the supply of the heating modules and disconnecting the equipment.

Another alarm corresponds to a low liquid level. If the equipment detects a low level in any of the fluid-filled reservoirs, it alerts by activating the low liquid level alarm, sending a message to the screen and giving off an intermittent beep, not allowing the treatment to start until the problem has been solved.

Additionally, the inclusion of an end-of-treatment alarm has been envisaged, in such a manner that upon conclusion of the treatment, the equipment sends a message to the screen indicating that the treatment has concluded.

Finally, an alarm indicating excessive cold or hot application or improper use of the equipment has been envisaged, although the equipment automatically controls application duration and does not allow a duration longer than the so-called safety duration to be programmed or operations that could place the treatment at risk to be executed, such as performing cold application without having previously carried out a hot application, etc. Even so, the equipment has an additional protection system that detects incorrect use thereof which could give rise to excessive cold or hot application, or any other action that places the treatment at risk. If this occurs, the equipment sends a message to the screen alerting of the error committed and prevents the programmed action from being carried out.

Going back to the contrast modules (17) again, said modules, the dimensions of which differ depending on the area or areas to be treated, cover the patient's treatment surface or surfaces and are made of impermeable, flexible and heat-conducting material wherethrough the fluid circulates at the preset temperature. The contrast modules are in contact with the patient (they are applied on a disposable plastic film for individual use and disposed between the contrast modules and the patient's skin for hygiene reasons). The contrast modules are in charge of transmitting the contrasting applications to the patient.

The contrast modules are joined to the thermostat control module (5) and, if applicable, therebetween, by means of the fluid supply and return pipes (43).

The contrast modules which can be used by the invention differ in size and shape, and are especially designed to suit the patient's needs and the surface to be treated in each case. For the description of the invention the following contrast modules have been embodied:
- 2 arm modules (17a).
- 2 leg modules (17b).
- 1 abdomen and chest module (17c).
- 1 back and buttock module (17d).
- 1 facial and upper chest module (23).

Figure 9 clearly shows a standard contrast module (17), designed so as to have internal spiral-shaped channels (44) which may differ in size, number and shape. For the description of the invention the channels are spiral-shaped, have a diameter of 0.5 mm and a separation of 0.5 mm therebetween. The fluid penetrates in the contrast module through an anti-return joint nut (45) and the fluid circulates through the interior of the module through the aforementioned channels until reaching the outlet (46); this outlet has another anti-return joint nut and is used either for connection to another contrast module or to close the circuit, in which case the anti-return joint nut (47) of the contrast module would be connected. This connection is made using the connection nipple (48). When it is connected, closing the circuit, the fluid circulates inside the contrast module through a return conduit (57) to the outlet thereof through the anti-return joint nut (49), said modules having the corresponding fixation strips or means (56).

Optionally, the channels that line the interior of the contrast modules may or may not house springs made of stainless steel or any other material, formally and dimensionally appropriate, or any other adequate element or device to avoid obstruction thereof.

The inlets/outlets of the module are connected respectively to the fluid inlets/outlets of the equipment (5a, 5a1, 5b, 5b1, 5c, 5c1, 5d, 5d1, 5e and 5e1).

The configuration of the contrast modules may or may not include inlet/outlet (46 and 47), i.e. they may be closed modules wherein the fluid flows into inlet (45) and flows out of outlet (49), without the possibility of interconnection to other contrast modules, or may be like that shown in figure 9, with interconnection points to other modules.

Connection between the different modules of the invention admits any configuration. Said interconnection between modules would be carried out in accordance with those areas of the patient's body that will be subjected to treatment. A configuration has been used for the description of the invention wherein, in order to perform a complete treatment, i.e. arms, legs, abdomen and back, in addition to a facial treatment and one with the direct contrasting applicator, the configuration used would be that represented in figure 1.

For the purpose of balancing the contrasting application times, flow reducers will be installed in the outlet corresponding to the back module (17d), in such a manner that the arm and other body applications have a similar duration.

With regard to the direct contrasting applicator (24), a detailed view of which is shown in figure 10, it is a manual device, especially designed for localised treatments in small areas (no more than 12 cm²). This device may differ in shape and size depending on the surface to be treated. It can be made of any material having sufficient heat-conducting characteristics. For the description of the invention a cylindrical shape has been chosen, made of aluminium or stainless steel and internally lined by a circuit (50) wherethrough the fluid that thermostatically controls the device in the contact area (51) thereof with the patient circulates, defining two channels in said circuit: an inlet channel and an outlet channel, while its base or application zone is formed by a hollow chamber irrigated throughout its surface by the circulating fluid.

Going back to the fluid inlets/outlets of the equipment again, said equipment incorporates an unspecified number of fluid inlets/outlets. The following inlets/outlets have been used in the description of the invention:
○ Inlets/outlets 5a and 5a1: supply and return of the left leg and back-buttocks contrast modules.
○ Inlets/outlets 5b and 5b1: supply and return of the right leg and abdomen-chest contrast modules.
○ Inlets/outlets 5c and 5c1: supply and return of the right arm and left arm contrast modules (the supply outlets of both arms are internally joined together and the return inlets of both arms are also internally joined together).
○ Inlets/outlets 5d and 5d1: supply and return of the facial module.
○ Inlets/outlets 5e and 5e1: supply and return of the direct contrasting applicator.

The different fluid inlets/outlets of the equipment, for the purpose of optimising the effect of the contrasting applications, may function sequentially and progressively. This operation is completely automatic and is implemented in the system's programming parameters, which allow adaptation of the equipment, on one hand, to the type of treatment to be performed and, on the other, to the morphology of the patient to be treated.

Both the inlet for facial applications (5d) and the direct contrasting applicator inlet (5e) act independently.

By way of a safety device and for the purpose of making the treatment more pleasant, without reducing the effectiveness of the contrast applications, the equipment, in those procedures where the treatment of legs and/or arms is combined with the treatment of the abdomen, automatically establishes the temperature, duration and frequency parameters, in such a manner that, without losing effectiveness, reduces the impact of cold application in more sensitive areas.

Likewise, the equipment automatically controls the safety margins of both the "initial hot application" and "initial cold application". These temperature curve, duration and sequence parameters are essential for guaranteeing, on one hand, the effectiveness and safety of the treatments and, on the other, not less importantly, the preparation of the patient for the treatment, with the (initial hot application), and essential for completing the treatment to ensure the patient's feeling of well-being and "energisation" upon concluding the process, with (final cold application).

As can be observed in figure 2, the equipment may be integrated in a bed (52) or constitute an independent element, having a hot fluid outlet (53) and cold fluid outlet (54) with access to the corresponding hot/cold fluid reservoirs.

Finally, with regard to operating modes, the invention has two basic operating modes:
- Manual operation.
- Automatic operation.

In manual operating mode the professional can perform, among others, functions such as:
- Through a user-friendly menu, he/she can select one of the standard beauty treatments: facial and/or body, or other treatments: shape-up, hydrotherapy, etc.
- Retrieve necessary data from the data base, in addition to the treatment history of a specific patient, either to verify said information or modify or add to it.
- Use the "direct contrasting applicator" simultaneously with the treatment being performed, in addition to other programmed functions.

The automatic operating mode is controlled by the control and programming module (2), which includes a microprocessor (58) and a power stage (55) for feeding the heating resistors (12), said control and programming module being designed for controlling purposes and for providing the professional with a flexible and powerful tool that is extraordinarily accurate and safe.

In the automatic operating mode the professional can perform functions such as:
- Through a user-friendly menu, he/she can select one of the standard beauty treatments: facial and/or body, or other treatments: shape-up, hydrotherapy, etc.
- Retrieve necessary data from the data base, in addition to the treatment history of a specific patient, either to verify said information or modify or add to it.
- Use the "direct contrasting applicator" simultaneously with the treatment being performed, in addition to other programmed functions.
- Among other, pre-programmed functions.

The treatments that can be selected in automatic operating mode include facial, body, shape-up and hydrotherapy, mud therapy with contrasting applications, etc.

Both in manual and automatic operating mode, the equipment provides operating data and parameters such as the following at all times:
- Status of the equipment: manual or automatic operating mode.
- Type of treatment being performed.
- Programmed treatment areas.
- Temperature ranges that we can use and selected temperature ranges.
- Number of programmed contrasting applications and those performed to date.
- Duration of the programmed contrasting applications, in addition to the time elapsed and that remaining in the application being performed at the time.
- Inlet(s)/outlet(s) which are being used at a given time, in addition to the temperature of the fluid in real time and in each of the inlets/outlets being used.
- Temperature of the direct contrasting applicator.
- Duration of the treatment, time at which the treatment started and time remaining for conclusion thereof.
- Patient's name and necessary details (previously introduced by the professional). (This data may be sent to the printer port to be used by the professional by way of patient record, etc.).
- According to type of treatment being performed, the equipment suggests the number of recommended treatments and the frequency with which they should be performed in order to obtain the best result. Likewise, it recommends a series of basic products that the patient should use at home to continue and consolidate the cabin treatment, in addition to elemental recommendations to be followed until the next treatment. (Although these data are programmed in-factory, the equipment allows the professional to complete and add to certain recommendation fields). (This information may be sent to the printer port).
- Data relative to the status and operation of the music module (CD player), among other others previously programmed .

## Claims

1. Device for performing beauty, physiotherapy and hydrotherapy treatments which, being especially intended for performing contrasting applications, i.e, cold and hot, rapid passage of heat to cold and vice versa, on any part of the body, either locally or generally, wherein the patient does not come into contact with the heat transfer vapours or fluids, the device comprising a casing (1) that houses a control and programming module (2), which in turn includes an alarms module (3), a music module (4), and a thermostat control module (5) with its corresponding fluid inlets (5a, 5a1, 5b, 5b1, 5c, 5c1, 5d, 5d1, 5e and 5e1), said control and programming module (2) including an inlet (10) for a touch screen (6) and a second inlet (11) for an external printer (7), wherein the thermostat control module (5) includes a cold module including one, two, three or more independent cold circuits (8) and a heat module including one, two, three or more heat circuits (9), both modules being aided by electrovalves (21a-n), wherein each heat circuit comprises heating resistors (12), while each cold circuit comprises Peltier Effect cells (27) or a conventional cold compressor, wherein a plurality of contrast modules (17) is selectively coupled through a series of fluid supply and return pipes (43) to the thermostat control module (5), wherethrough the fluid previously treated by the thermostat control module (5) flows, wherein the contrast modules (17), formally and dimensionally appropriate to a body part to be treated, such as arms, legs, chest, back, buttocks, face or areas having a small surface, are made of an impermeable, flexible and heat-conducting material, wherein the programming module incorporates programming software for controlling the supply to the different contrast modules (17) depending on the area to be treated, the type of treatment, duration thereof, the frequency between applications and the specific temperature of each application, wherein the contrast modules (17) include a series of spiral-shaped internal channels (44) which can differ in shape, number and size, whereby the fluid penetrates in the contrast modules through an anti-return joint nut (45) up to the outlet (46), wherein another anti-return joint nut is disposed, which incorporates a return conduit (57) finishing at its ends in corresponding anti-return joint nuts (47) and (49), said fluid inflow/outflow joint nuts of the conduits (57) and internal channels (44) being elements that constitute connecting means for connecting to other contrast modules or connecting means for connecting to the pipes (43), wherein said modules can be complemented by a connection nipple (48) for closing the circuit, wherein the heat module, which is equipped with electrovalves (21), additionally comprises elements common to the different independent heat circuits, such as a hot fluid reservoir (15), a radiator (19), a heating resistor module (12) and a heat pump (18), whereby each of the independent heat circuits comprises a heating resistor module (12), a discharge pump (22a, 22b, 22c) and independent temperature sensors (16c, 16d, 16e), while the cold module, which is also equipped with electrovalves (21), incorporates elements common to the different independent cold circuits, such as a cold fluid reservoir (20), a high-efficency cooling module (13) and a cold pump (14), and whereby each of the independent circuits comprises a discharge pump (22a, 22b, 22c) and the corresponding independent temperature sensors (16c, 16d, 16e).

2. Device for performing beauty, physiotherapy and hydrotherapy treatments, according to claim 1, **characterised in that** the channels that line the interior of the contrast modules incorporate anti-obstruction means such as springs made of stainless steel or any other material or similar.

3. Device for performing beauty, physiotherapy and hydrotherapy treatments, according to claim 1, **characterised in that** the different fluid inlets/ incorporate flow reducers, in such a manner as to adapt the flow of each inlet to the volume of fluid housed inside the corresponding exchanger.

4. Device for performing beauty, physiotherapy and hydrotherapy treatments, according to claim 1, **characterised in that** the equipment incorporates pressure sensors.

5. Device for performing beauty, physiotherapy and hydrotherapy treatments, according to claim 1, **characterised in that** both the heat and cold modules incorporate elements common to the respective heat circuits and cold circuits that supply the contrast modules (17).

6. Device for performing beauty, physiotherapy and hydrotherapy treatments, according to claim 1, **characterised in that** the cold module incorporates elements common to the different independent cold circuits, such as a cold fluid reservoir (20), a high-efficiency cocling module (13) and a cold pump (14), and that each of the independent cold circuits comprises a discharge pump (22a, 22b, 22c) and independent temperature sensors (16c, 16d, 16e).

7. Device for performing beauty, physiotherapy and hydrotherapy treatments, according to the preceding claims, **characterised in that** each heat circuit comprises heating resistor module (12), a discharge pump (22a, 22b, 22c) and independent temperature sensors (16c, 16d, 16e).

8. Device for performing beauty, physiotherapy and hydrotherapy treatments, according to claim 6, **characterised in that** the high-efficiency cooling module (13) is based on Peltier Effect cells (27), having a circulator unit disposed therein, wherethrough the fluid to be cooled circulates and is cooled by means of Peltier Effect cells (27), the circulator unit being formed by at least one circulator element (25) made of heat-conducting material, machined internally in a spiral shape, element wherewith a series of Peltier cells (27) are associated, equipped with means for reversing their polarity, cells which may be conventional or double and which may be connected serially or in parallel.

9. Device for performing beauty, physiotherapy and hydrotherapy treatments, according to claim 8, **characterised in that** their respective radiators (42), whereon complementary fans (30) are disposed, the radiators and fans forming heat exchangers.

10. Device for performing beauty, physiotherapy and hydrotherapy treatments, according to claims 8 and 9,
**characterised in that** the system for fixing the Peltier cell (27), on one hand, to its corresponding separator and to the circulator and, on the other, to its own heat exchanger, is composed of three screws per circulator (25), in such a manner that they pass through the corresponding circulator using, for such purpose, isolators that prevent the screws from coming into contact with the circulator, said screws passing through the circulator through three drill holes (31) made therein and being fixed by nuts to the base of the respective radiators (19).

11. Device for performing beauty, physiotherapy and hydrotherapy treatments, according to claims 9 to 10, **characterised in that** the circulators (25) are susceptible to incorporating a turbulator therein.

12. Device for performing beauty, physiotherapy and hydrotherapy treatments, according to claims 9 to 11, **characterised in that** the heat exchangers of the circulators are housed in a box made of sheet metal or any other material and insulated from the exterior and therebetween by an insulating material.

13. Device for performing beauty, physiotherapy and hydrotherapy treatments, according to claims 1 and 2, **characterised in that** the device incorporates a direct contrast applicator (24), materialised in a manual device, especially designed for local treatments in small areas,
preferably cylindrical, made of aluminum or stainless steel, the interior of which is lined by a circuit wherethrough the fluid flows to control the temperature of the device in the area that is in contact with the patient, whereby at the base or application area of the device there is a hollow chamber that is irrigated throughout its surface by the circulating fluid.

14. Device for performing beauty, physiotherapy and hydrotherapy treatments, according to claim 8, **characterised in that** the Peltier cells (27), on their cold side, are installed back to back on each of the sides of the circulator modules that form the circulator unit, while connection thereof to the aforementioned circulators is carried out by means of separators (28) made of heat-conducting material, while said Peltier cells (27) are joined, on their hot side, by means of heat-conducting paste to a fluid-based cooling system.

15. Device for performing beauty, physiotherapy and hydrotherapy treatments, according to claim 1, **characterised in that** the device is integrated in a bed.

## Patentansprüche

1. Vorrichtung zur Durchführung von Schönheits-, Physiotherapie- und Hydrotherapiebehandlungen, die insbesondere dazu vorgesehen ist, kontrastierende Anwendungen, d. h. kalt und heiß, mit einem schnellen Wechsel zwischen Hitze und Kälte und umgekehrt, an einem beliebigen Körperteil entweder lokal oder allgemein durchzuführen, wobei der Patient nicht in Kontakt mit den die Wärme übertragenden Dämpfen oder Fluiden kommt, wobei die Vorrichtung ein Gehäuse (1) umfasst, in das ein Steuerungs- und Programmiermodul (2) aufgenommen ist, das seinerseits ein Alarmmodul (3), ein Musikmodul (4) und ein Thermostatregelmodul (5) mit entsprechenden Fluideinlässen (5a, 5a1, 5b, 5b1, 5c, 5c1, 5d, 5d1, 5e und 5e1) einschließt, wobei das besagte Steuerungs- und Programmiermodul (2) einen Eingang (10) für einen Touchscreen (6) und einen zweiten Eingang (11) für einen externen Drucker (7) umfasst, wobei das Thermostatregelmodul (5) ein Kältemodul mit einem, zwei, drei oder mehr unabhängigen Kältekreisläufen (8) und ein Wärmemodul mit einem, zwei, drei oder mehr Wärmekreisläufen (9) umfasst, wobei beide Module von Elektroventilen (21a-n) unterstützt werden, wobei jeder Wärmekreislauf Heizwiderstände (12) einschließt, während jeder Kältekreislauf Peltier-Effekt-Zellen (27) oder einen herkömmlichen Kältekompressor umfasst, wobei eine Vielzahl von Kontrastmodulen (17) über eine Reihe von Fluid-Zufuhr- und Rückführrohrleitungen (43) selektiv mit dem Thermostatregelmodul (5) gekoppelt ist, durch die das vorher von dem Thermostatregelmodul (5) behandelte Fluid fließt, wobei die Kontrastmodule (17), die von ihrer Form und ihren Abmessungen her für einen zu behandelnden Körperteil wie Arme, Beine, Brust, Rücken, Gesäß, Gesicht oder Bereiche mit einer kleinen Oberfläche geeignet sind, aus einem undurchlässigen, flexiblen und Wärme leitenden Material gefertigt sind, wobei das Programmiermodul eine Programmiersoftware zur Steuerung der Versorgung der unterschiedlichen Kontrastmodule (17) einschließt, abhängig von dem zu behandelnden Bereich, der Behandlungsart, der Dauer derselben, der Häufigkeit zwischen Anwendungen und der spezifischen Temperatur jeder Anwendung, wobei die Kontrastmodule (17) eine Reihe von spiralförmigen Innenkanälen (44) einschließen, die eine unterschiedliche Form, Anzahl und Größe besitzen können, wobei das Fluid über eine Rückschlag-Verbindungsmutter (45) bis an den Auslass (46) in die Kontrastmodule eindringt, wobei eine andere Rückschlag-Verbindungsmutter vorgesehen ist, in die eine Rückführleitung (57) eingeschlossen ist, die an ihren Enden in entsprechenden Rückschlag-Verbindungsmuttern (47) und (49) endet, wobei die besagten Fluid-Einlass-/Auslassverbindungsmuttern der Leitungen (57) und der Innenkanäle (44) Elemente sind, die Verbindungsmittel zum Anschluss an andere Kontrastmodule oder Verbindungsmittel zum Anschluss an die Rohrleitungen (43) bilden, wobei die besagten Module mit einem Verbindungsnippel (48) zwecks Verschluss des Kreislaufs ergänzt werden können, wobei das Wärmemodul, das mit Elektroventilen (21) ausgestattet ist, zusätzlich Elemente umfasst, die den unterschiedlichen unabhängigen Wärmekreisläufen gemeinsam sind, wie beispielsweise einen Behälter für das heiße Fluid (15), einen Heizkörper (19), ein Heizwiderstandsmodul (12) und eine Wärmepumpe (18), wobei jeder der unabhängigen Wärmekreisläufe ein Heizwiderstandsmodul (12), eine Ablaufpumpe (22a, 22b, 22c) und unabhängige Temperaturfühler (16c, 16d, 16e) umfasst, während das Kältemodul, das ebenfalls mit Elektroventilen (21) ausgestattet ist, Elemente einschließt, die den unterschiedlichen unabhängigen Kältekreisläufen gemeinsam sind, wie beispielsweise einen Behälter für das kalte Fluid (20), ein hocheffizientes Kühlmodul (13) und eine Kältepumpe (14), und wobei jeder der unabhängigen Kreisläufe eine Ablaufpumpe (22a, 22b, 22c) und die entsprechenden unabhängigen Temperaturfühler (16c, 16d, 16e) umfasst.

2. Vorrichtung zur Durchführung von Schönheits-, Physiotherapie- und Hydrotherapiebehandlungen nach Anspruch 1, **dadurch gekennzeichnet, dass** die Kanäle, die das Innere der Kontrastmodule auskleiden, Verstopfungsschutzmittel wie beispielsweise Federn einschließen, die aus Edelstahl oder einem anderen oder ähnlichen Material gefertigt sind.

3. Vorrichtung zur Durchführung von Schönheits-, Physiotherapie- und Hydrotherapiebehandlungen nach Anspruch 1, **dadurch gekennzeichnet, dass** die unterschiedlichen Fluideinlässe Durchflussbegrenzer in einer Weise einschließen, die es ermöglicht, den Durchfluss jedes Einlasses an das Fluidvolumen anzupassen, das innerhalb des entsprechenden Austauschers enthalten ist.

4. Vorrichtung zur Durchführung von Schönheits-, Physiotherapie- und Hydrotherapiebehandlungen nach Anspruch 1, **dadurch gekennzeichnet, dass** die Ausstattung Drucksensoren einschließt.

5. Vorrichtung zur Durchführung von Schönheits-, Physiotherapie- und Hydrotherapiebehandlungen nach Anspruch 1, **dadurch gekennzeichnet, dass** sowohl die Wärmeals auch die Kältemodule Elemente einschließen, die den entsprechenden Wärme- und Kältekreisläufen gemeinsam sind, welche die Kontrastmodule (17) versorgen.

6. Vorrichtung zur Durchführung von Schönheits-, Physiotherapie- und Hydrotherapiebehandlungen nach Anspruch 1, **dadurch gekennzeichnet, dass** das Kältemodul Elemente einschließt, die den unterschiedlichen unabhängigen Kältekreisläufen gemeinsam sind, wie beispielsweise einen Behälter für das kalte Fluid (20), ein hocheffizientes Kühlmodul (13) und eine Kältepumpe (14) und dass jeder der unabhängigen Kältekreisläufe eine Ablaufpumpe (22a, 22b, 22c) und unabhängige Temperaturfühler (16c, 16d, 16e) umfasst.

7. Vorrichtung zur Durchführung von Schönheits-, Physiotherapie- und Hydrotherapiebehandlungen nach den vorherigen Ansprüchen, **dadurch gekennzeichnet, dass** jeder Wärmekreislauf ein Heizwiderstandsmodul (12), eine Ablaufpumpe (22a, 22b, 22c) und unabhängige Temperaturfühler (16c, 16d, 16e) umfasst.

8. Vorrichtung zur Durchführung von Schönheits-, Physiotherapie- und Hydrotherapiebehandlungen nach Anspruch 6, **dadurch gekennzeichnet, dass** das hocheffiziente Kühlmodul (13) auf Peltier-Effekt-Zellen (27) beruht, in denen eine Umwälzeinheit angeordnet ist, durch die das zu kühlende Fluid zirkuliert und von Peltier-Effekt-Zellen (27) abgekühlt wird, wobei die Umwälzeinheit von mindestens einem Umwälzelement (25) gebildet wird, das aus Wärme leitendem Material besteht und innen spanabhebend in einer Spiralform bearbeitet ist, ein Element, mit dem eine Reihe von Peltier-Zellen (27) verknüpft sind, die mit Mitteln zur Umkehrung deren Polarität ausgestattet sind, wobei diese Zellen herkömmlich oder doppelt sowie in Reihe oder parallel geschaltet sein können.

9. Vorrichtung zur Durchführung von Schönheits-, Physiotherapie- und Hydrotherapiebehandlungen nach Anspruch 8, **dadurch gekennzeichnet, dass** die Peltier-Zellen (27) an ihrer kalten Seite Rücken an Rücken an jeder der Seiten der Umwälzmodule installiert sind, welche die Umwälzeinheit bilden, während die Verbindung derselben mit den vorgenannten Umwälzelementen mittels Abstandsstücken (28) durchgeführt wird, die aus Wärme leitendem Material gefertigt sind, während die besagten Peltier-Zellen (27) an ihrer warmen Seite mittels einer Wärme leitenden Paste mit deren entsprechenden Heizkörpern (42) verbunden sind, auf denen ergänzende Lüfter (30) angeordnet sind, wobei die Heizkörper und Lüfter Wärmeaustauscher bilden.

10. Vorrichtung zur Durchführung von Schönheits-, Physiotherapie- und Hydrotherapiebehandlungen nach Anspruch 8 und 9, **dadurch gekennzeichnet, dass** das System, um die Peltier-Zelle (27) auf der einen Seite an deren entsprechendem Abstandsstück und dem Umwälzelement und auf der anderen Seite an deren eigenem Wärmeaustauscher zu befestigen, aus drei Schrauben pro Umwälzelement (25) in einer Weise besteht, die es ermöglicht, dass diese das entsprechende Umwälzelement passieren, wobei zu diesem Zweck Isolatoren verwendet werden, die verhindern, dass die Schrauben in Kontakt mit dem Umwälzelement kommen, wobei die besagten Schrauben das Umwälzelement durch drei in diesem vorgesehene Bohrungen (31) passieren und mit Muttern am Boden der entsprechenden Heizkörper (19) befestigt sind.

11. Vorrichtung zur Durchführung von Schönheits-, Physiotherapie- und Hydrotherapiebehandlungen nach Anspruch 9 bis 10, **dadurch gekennzeichnet, dass** die Umwälzelemente (25) einen darin eingefügten Turbulator einschließen können.

12. Vorrichtung zur Durchführung von Schönheits-, Physiotherapie- und Hydrotherapiebehandlungen nach Anspruch 9 bis 11, **dadurch gekennzeichnet, dass** die Wärmeaustauscher der Umwälzelemente in einem Gehäuse aus Blech oder einem anderen Material aufgenommen und nach außen und im Zwischenraum mit einem Isoliermaterial isoliert sind.

13. Vorrichtung zur Durchführung von Schönheits-, Physiotherapie- und Hydrotherapiebehandlungen nach Anspruch 1 und 2, **dadurch gekennzeichnet, dass** die Vorrichtung einen direkten Kontrastapplikator (24) einschließt, der in einer manuellen, speziell zur lokalen Behandlung von kleinen Bereichen ausgelegten, vorzugsweise zylinderförmigen Vorrichtung ausgeführt ist, die aus Aluminium oder Edelstahl gefertigt und innen mit einem Kreislauf ausgekleidet ist, durch den das Fluid fließt, um die Temperatur der Vorrichtung in dem Bereich zu regeln, der in Kontakt mit dem Patienten ist, wobei am Boden oder dem Anwendungsbereich der Vorrichtung ein Hohlraum vorhanden ist, dessen gesamte Oberfläche von dem zirkulierenden Fluid beregnet wird.

14. Vorrichtung zur Durchführung von Schönheits-, Physiotherapie- und Hydrotherapiebehandlungen nach Anspruch 8, **dadurch gekennzeichnet, dass** die Peltier-Zellen (27) an ihrer kalten Seite Rücken an Rücken an jeder der Seiten der Umwälzmodule installiert sind, welche die Umwälzeinheit bilden, während die Verbindung derselben mit den vorgenannten Umwälzelementen mittels Abstandsstücken (28) durchgeführt wird, die aus Wärme leitendem Material gefertigt sind, während die besagten Peltier-Zellen (27) an ihrer warmen Seite mittels einer Wärme leitenden Paste mit einem auf einem Fluid beruhenden Kühlsystem verbunden sind.

15. Vorrichtung zur Durchführung von Schönheits-, Physiotherapie- und Hydrotherapiebehandlungen nach Anspruch 1, **dadurch gekennzeichnet, dass** die Vorrichtung in einem Bett integriert ist.

## Revendications

1. Dispositif pour procéder à des traitements esthétiques, physiothérapiques et hydrothérapiques qui, étant destiné, en particulier, à la réalisation d'applications contrastées, c'est-à-dire froid et chaud, passage rapide de la chaleur au froid et vice versa, sur n'importe quelle partie du corps, soit de façon locale soit de façon générale, où le patient n'est pas en contact avec les vapeurs ou les fluides de transfert de chaleur, le dispositif comprenant un boîtier (1) qui héberge un module de commande et de programmation (2), qui à son tour comprend un module alarmes (3), un module musique (4), et un module de commande de thermostat (5) avec ses entrées de fluide correspondantes (5a, 5a1, 5b, 5b1, 5c, 5c1, 5d, 5d1, 5e et 5e1), ledit module de commande et de programmation (2) comprenant une entrée (10) pour un écran tactile (6) et une seconde entrée (11) pour une imprimante externe (7), où le module de commande de thermostat (5) comprend un module froid comportant un, deux, trois ou plusieurs circuits de froid indépendants (8) et un module chaleur comportant un, deux, trois ou plusieurs circuits de chaleur (9), les deux modules étant assistés par des électrovalves (21a-n), où chaque circuit de chaleur comprend des résistances de chauffage (12), tandis que chaque circuit de froid comprend des cellules à effet Peltier (27) ou un compresseur de froid conventionnel, module de commande de thermostat (5), où une pluralité de modules de contraste (17) est couplée de manière sélective via une série de tuyaux d'alimentation en fluide et de retour de fluide (43) au module de commande de thermostat (5) à travers lesquels circule le fluide préalablement traité par le module de commande de thermostat (5), où les modules de contraste (17) aux formes et aux dimensions adaptées à une partie du corps à traiter, telle que bras, jambes, poitrine, dos, fesses, visage ou zones de petite surface, sont fabriqués dans un matériau imperméable, souple et conducteur de chaleur, où le module de programmation comprend un logiciel de programmation pour commander l'alimentation aux différents modules de contraste (17) en fonction de la zone à traiter, du type de traitement, de sa durée, de la durée entre les applications et de la température spécifique de chaque application, où les modules de contraste (17) comprennent une série de canaux internes en forme de spirale (44) dont la forme, le nombre et la taille peuvent être différents, par lequel le fluide pénètre dans les modules de contraste via un écrou d'assemblage anti-retour (45) jusqu'à la sortie (46), où un autre écrou d'assemblage anti-retour est installé, qui comporte un conduit retour (57) finissant à ses extrémités en écrous d'assemblage anti-retour associés (47) et (49), lesdits écrous d'assemblage d'arrivée/évacuation de fluide des conduits (57) et canaux internes (44) étant des éléments qui constituent des moyens de raccordement pour le raccordement aux autres modules de contraste ou des moyens de raccordement pour le raccordement aux tuyaux (43), où lesdits modules peuvent être complétés par un raccord fileté (48) pour fermer le circuit, où le module chaleur, qui est équipé d'électrovalves (21), comprend en outre des éléments communs aux différents circuits de chaleur indépendants, comme un réservoir de fluide chaud (15), un radiateur (19), un module de résistances de chauffage (12) et une pompe à chaleur (18), par lequel chacun des circuits de chaleur indépendants comprend un module de résistances de chauffage (12), une pompe d'évacuation (22a, 22b, 22c) et des capteurs de température indépendants (16c, 16d, 16e), tandis que le module froid, qui est également équipé d'électrovalves (21), comprend des éléments communs aux différents circuits de froid indépendants, comme un réservoir de fluide froid (20), un module de refroidissement (13) de haute efficacité et une pompe à froid (14), et par lequel chacun des circuits indépendants comprend une pompe d'évacuation (22a, 22b, 22c) et les capteurs de température indépendants (16c, 16d, 16e) associés.

2. Dispositif pour procéder à des traitements esthétiques, physiothérapiques et hydrothérapiques, selon la revendication 1, **caractérisé en ce que** les canaux qui doublent l'intérieur des modules de contraste comprennent des moyens anti-obstruction comme des ressorts fabriqués en acier inoxydable ou en tout autre matériau ou des pièces similaires.

3. Dispositif pour procéder à des traitements esthétiques, physiothérapiques et hydrothérapiques, selon la revendication 1, **caractérisé en ce que** les différentes entrées de fluide/ comprennent des réducteurs de flux, de façon à adapter le flux de chaque entrée au volume de fluide logé à l'intérieur de l'échangeur associé.

4. Dispositif pour procéder à des traitements esthétiques, physiothérapiques et hydrothérapiques, selon la revendication 1, **caractérisé en ce que** l'appareil comprend des capteurs de pression.

5. Dispositif pour procéder à des traitements esthétiques, physiothérapiques et hydrothérapiques, selon la revendication 1, **caractérisé en ce que** les modules chaleur et froid comprennent tous deux des éléments communs aux circuits de chaleur et aux circuits de froid associés qui alimentent les modules de contraste (17).

6. Dispositif pour procéder à des traitements esthétiques, physiothérapiques et hydrothérapiques, selon la revendication 1, **caractérisé en ce que** le module froid comprend des éléments communs aux différents circuits de froid indépendants, comme un réservoir de fluide froid (20), un module de refroidissement (13) de haute efficacité et une pompe à froid (14), et **en ce que** chacun des circuits de froid indépendants comprend une pompe d'évacuation (22a, 22b, 22c) et des capteurs de température indépendants (16c, 16d, 16e).

7. Dispositif pour procéder à des traitements esthétiques, physiothérapiques et hydrothérapiques, selon les revendications précédentes, **caractérisé en ce que** chaque circuit de chaleur comprend un module de résistances de chauffage (12), une pompe d'évacuation (22a, 22b, 22c) et des capteurs de température indépendants (16c, 16d, 16e).

8. Dispositif pour procéder à des traitements esthétiques, physiothérapiques et hydrothérapiques, selon la revendication 6, **caractérisé en ce que** le module de refroidissement (13) de haute efficacité est basé sur des cellules à effet Peltier (27), une unité de circulateur y étant disposée, à travers laquelle le fluide à refroidir circule et est refroidi au moyen de cellules à effet Peltier (27), l'unité de circulateur étant formée par au moins un élément de circulateur (25) fabriqué dans un matériau conducteur de chaleur, usiné intérieurement en forme de spirale, élément auquel une série de cellules Peltier (27) sont associées, équipées de moyens pour inverser leur polarité, cellules qui peuvent être conventionnelles ou doubles et qui peuvent être connectées en série ou en parallèle.

9. Dispositif pour procéder à des traitements esthétiques, physiothérapiques et hydrothérapiques, selon la revendication 8, **caractérisé en ce que** les cellules Peltier (27), sur leur côté froid, sont installées dos à dos sur chacun des côtés des modules de circulateur qui forment l'unité de circulateur, tandis que leur connexion aux circulateurs mentionnés ci-dessus est réalisée au moyen de séparateurs (28) fabriqués dans un matériau conducteur de chaleur, tandis que lesdites cellules Peltier (27) sont assemblées, sur leur côté chaud, au moyen de colle conductrice de chaleur à leurs radiateurs respectifs (42), sur lesquels des ventilateurs complémentaires (30) sont installés, les radiateurs et les ventilateurs formant des échangeurs de chaleur.

10. Dispositif pour procéder à des traitements esthétiques, physiothérapiques et hydrothérapiques, selon les revendications 8 et 9, **caractérisé en ce que** le système pour fixer la cellule Peltier (27), d'une part, à son séparateur associé et au circulateur et, d'autre part, à son propre échangeur de chaleur, se compose de trois vis par circulateur (25), de façon à ce qu'elles passent à travers le circulateur associé en utilisant, pour ce faire, des isolateurs qui empêchent les vis d'entrer en contact avec le circulateur ; lesdites vis traversant le circulateur via trois trous forés (31) qui y sont faits et étant fixées par des écrous à la base des radiateurs respectifs (19).

11. Dispositif pour procéder à des traitements esthétiques, physiothérapiques et hydrothérapiques, selon les revendications 9 à 10, **caractérisé en ce que** les circulateurs (25) ont susceptibles de comporter au-dedans un turbulateur.

12. Dispositif pour procéder à des traitements esthétiques, physiothérapiques et hydrothérapiques, selon les revendications 9 à 11, **caractérisé en ce que** les échangeurs de chaleur des circulateurs sont logés dans une boîte fabriquée dans une feuille de métal ou dans tout autre matériau et isolés de l'extérieur et entre eux par un matériau isolant.

13. Dispositif pour procéder à des traitements esthétiques, physiothérapiques et hydrothérapiques, selon les revendications 1 et 2, **caractérisé en ce que** le dispositif comprend un applicateur de contraste direct (24), matérialisé en un dispositif manuel, spécialement conçu pour les traitements locaux dans de petites zones, de préférence cylindrique, fabriqué en aluminium ou en acier inoxydable, dont l'intérieur est doublé par un circuit à travers lequel le fluide circule pour contrôler la température du dispositif dans la zone qui est en contact avec le patient, au moyen duquel à la base ou dans la zone d'application du dispositif il y a une chambre creuse qui est irriguée sur toute sa surface par le fluide en circulation.

14. Dispositif pour procéder à des traitements esthétiques, physiothérapiques et hydrothérapiques, selon la revendication 8, **caractérisé en ce que** les cellules Peltier (27), sur leur côté froid, sont installées dos à dos sur chacun des côtés des modules de circulateur qui forment l'unité de circulateur, tandis que leur connexion aux circulateurs mentionnés ci-dessus est réalisée au moyen de séparateurs (28) fabriqués dans un matériau conducteur de chaleur, tandis que lesdites cellules Peltier (27) sont assemblées, sur leur côté chaud, au moyen de colle conductrice de chaleur à un système de refroidissement à base de fluide.

15. Dispositif pour procéder à des traitements esthétiques, physiothérapiques et hydrothérapiques, selon la revendication 1, **caractérisé en ce que** le dispositif est intégré dans un banc.
